# EUROPEAN PATENT APPLICATION

(11) **EP 4 717 763 A1**
(43) Date of publication of application: **01.04.2026**
(21) Application number: 25802841.4
(22) Date of filing: 09.05.2025
(51) Int. Cl.: C12N 1/20, A61K 35/745, A61K 35/747, A61P 19/02, A61P 29/00, A61P 19/06, C12R 1/01, C12R 1/225

(54) **COMPOUND PROBIOTIC CAPABLE OF ALLEVIATING GOUTY ARTHRITIS AND USE THEREOF**

(30) Priority: 11.05.2024 CN 202410579172
(71) Applicant: Wecare Probiotics Co., Ltd., Suzhou, Jiangsu 215200 (CN)
(72) Inventor: FANG, Shuguang, Suzhou, Jiangsu 215200 (CN); FAN, Yixuan, Suzhou, Jiangsu 215200 (CN); GU, Jiayue, Suzhou, Jiangsu 215200 (CN); QI, Yongmei, Suzhou, Jiangsu 215200 (CN); GAI, Zhonghui, Suzhou, Jiangsu 215200 (CN); ZHU, Jianguo, Suzhou, Jiangsu 215200 (CN)
(74) Representative: Prinz & Partner mbB
(86) International application number: PCT/CN2025/093796
(87) International publication number: WO 2025/237192

(57) **Abstract**

The present application provides a compound probiotic capable of alleviating gouty arthritis and the use thereof. The compound probiotic capable of alleviating gouty arthritis consists of a Bifidobacterium longum BL21 strain and a Lactobacillus salivarius LS97 strain. The present application has discovered that the two strains can work together, mutually promote each other, and exhibit a synergistic effect in alleviating gouty arthritis. The synergistic effect is specifically demonstrated by the following: the ability to reduce pain-sensitive reactivity; the ability to decrease the number of white blood cells and the aggregation of neutrophils in joints; the ability to lower the activity of myeloperoxidase in periarticular tissues; the ability to reduce the production of the inflammatory factors CXCL1 and IL-1β; and the ability to increase the expression level of the anti-inflammatory cytokine IL-10 in joint tissues.

## Description

### TECHNICAL FIELD

The present application belongs to the technical field of probiotics and relates to a composite probiotic for alleviating gouty arthritis and a use thereof.

### BACKGROUND

Gout is a disease that seriously impacts joint health, and the pathogenesis of gout is closely related to uric acid metabolism disorder. When uric acid level in a patient's body rises, uric acid crystals deposit in the joints and surrounding tissues, triggering intense pain and inflammatory responses. These uric acid crystals accumulate in joints, especially in joint parts such as toes, ankles, knees and fingers, causing the attack of acute arthritis. Patients may experience sudden pain and swelling, and even a slight touch may trigger severe pain. In addition to acute gouty arthritis, gout may also cause chronic arthritis, and long-term uric acid deposition may form tophi around joints, affecting joint functions.

Therefore, gout is not only a joint disease, but also a disease that has a serious effect on the overall health and quality of life of patients. Timely diagnosis and treatment are crucial to alleviate symptoms and prevent recurrence. Probiotics can promote the balance of beneficial flora in the intestinal tract, which may help improve the metabolism of uric acid and reduce the generation of uric acid in the body or increase the excretion rate of uric acid. The imbalance of intestinal flora is related to the pathogenesis of gout. By maintaining the balance of intestinal flora, probiotics can reduce the growth of harmful bacteria and indirectly affect the metabolism of uric acid, which may have a positive effect on the prevention and alleviation of gout. Therefore, using probiotics to control the intestinal microbiota may be an effective treatment or preventive measure, which can improve hyperuricemia and alleviate the problems such as gout.

### SUMMARY

The present application provides a composite probiotic for alleviating gouty arthritis and a use thereof, and in particular, provides a composite probiotic for alleviating gouty arthritis, a probiotic preparation for alleviating gouty arthritis and a use of the composite probiotic or the probiotic preparation in the preparation of a product for preventing, alleviating or treating gouty arthritis.

In a first aspect, the present application provides a composite probiotic for alleviating gouty arthritis consisting of a *Bifidobacterium longum* BL21 strain with a deposit number CGMCC No. 10452 and a *Lactobacillus salivarius* LS97 strain with a deposit number CGMCC No. 16922.

The *Bifidobacterium longum* BL21 strain is deposited in the China General Microbiological Culture Collection Center on January 27, 2015, with a deposit number CGMCC No. 10452, where the China General Microbiological Culture Collection Center is located at No. 3, NO. 1 West Beichen Road, Chaoyang District, Beijing. The *Lactobacillus salivarius* LS97 strain is deposited in the China General Microbiological Culture Collection Center on December 10, 2018, with a deposit number CGMCC No. 16922, where the China General Microbiological Culture Collection Center is located at No. 3, NO. 1 West Beichen Road, Chaoyang District, Beijing.

The present application creatively develops a novel probiotic compounding manner and a novel strategy for preventing and treating gout, that is, the *Bifidobacterium longum* BL21 strain and the *Lactobacillus salivarius* LS97 strain are compounded. It is found that the two strains can cooperate with each other, promote each other and exert a synergistic effect in alleviating gouty arthritis, which is specifically manifested by: (1) reducing pain sensitivity; (2) reducing the number of leukocytes and the aggregation of neutrophils in joints; (3) reducing the activity of myeloperoxidase in periarticular tissues; (4) reducing the generation of inflammatory factors CXCL1 and IL-1β; and (5) improving the expression level of anti-inflammatory cytokines IL-10 in joint tissues.

In the case where amounts of bacteria used are consistent, compared with an intervention mode of a single BL21 strain or a single LS97 strain, the effect of the compounding of the two bacteria in alleviating and improving gouty arthritis is significantly improved. Therefore, the composite probiotic have a good prospect of being used for preparing the product for preventing, alleviating or treating gouty arthritis. Moreover, the two bacteria are both probiotics, and the product has high safety and is not easy to develop resistance.

Preferably, a ratio of the viable bacterial count of the BL21 strain to the viable bacterial count of the LS97 strain is 1:10 to 10:1, for example, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1 or 10:1. Other specific point values within the numerical range may be selected, and details are not described here.

In a second aspect, the present application provides a probiotic preparation for alleviating gouty arthritis. The probiotic preparation includes the composite probiotic of the first aspect.

Preferably, a total viable bacterial count in the probiotic preparation is not less than 1×10⁹ CFU/mL or 1×10⁹ CFU/g, for example, 1×10⁹ CFU/g (CFU/mL), 2×10⁹ CFU/g (CFU/mL), 5×10⁹ CFU/g (CFU/mL), 8×10⁹ CFU/g (CFU/mL), 1×10¹⁰ CFU/g (CFU/mL), 5×10¹⁰ CFU/g (CFU/mL), 1×10¹¹ CFU/g (CFU/mL), 1×10¹² CFU/g (CFU/mL) or 1×10¹³ CFU/g (CFU/mL). Other specific point values within the numerical range may be selected, and details are not described here.

Preferably, a dosage form of the probiotic preparation includes a lyophilized powder, a capsule, a tablet or a granules.

The dosage form of the probiotic preparation to which the present application relates is not limited and includes the most commonly used lyophilized powder, or further prepared capsules, tablets or granules.

Preferably, the dosage form of the probiotic preparation is the lyophilized powder, and the lyophilized powder is prepared through the following preparation method:
inoculating a BL21 strain and an LS97 strain in mediums, respectively, for activation and fermentation culture to obtain fermentation broths; separately centrifuging the fermentation broths, mixing with a lyoprotectant, and then performing lyophilization to obtain BL21 bacteria powder and LS97 bacteria powder; and mixing the BL21 bacteria powder with the LS97 bacteria powder according to a ratio of viable bacterial count to obtain the probiotic preparation for alleviating gouty arthritis.

Preferably, the lyoprotectant is selected from any one or a combination of at least two of skim milk, gelatin, dextrin, arabic gum, dextran, sodium alginate, polyvinylpyrrolidone, sucrose, lactose, trehalose, sorbitol or xylitol.

Further preferably, the BL21 bacteria powder and the LS97 bacteria powder are mixed according to the ratio of viable bacterial count and mixed with a functional additive.

The functional additive is selected from any one or a combination of at least two of fructooligosaccharides, galactooligosaccharides, xylooligosaccharides, isomaltooligosaccharides, soybean oligosaccharides, inulin, spirulina, *arthrospira,* Krestin, stachyose, polydextrose, α-whey protein or lactoferrin.

In a third aspect, the present application provides a use of the composite probiotic of the first aspect or the probiotic preparation of the second aspect in the preparation of a product for preventing, alleviating or treating gouty arthritis.

Preferably, the product further includes an adjuvant.

Preferably, the adjuvant is selected from any one or a combination of at least two of a filler, a binder, a wetting agent, a disintegrating agent, an emulsifier, a cosolvent, a solubilizer, an osmotic pressure regulator, a colorant, a pH adjusting agent, an anti-oxidant, a bacteriostatic agent or a buffer.

Compared with the existing art, the present application has the beneficial effects below.

The present application creatively develops a novel probiotic compounding manner and a novel strategy for preventing and treating gout, that is, the *Bifidobacterium longum* BL21 strain and the *Lactobacillus salivarius* LS97 strain are compounded. It is found that the two strains can cooperate with each other, promote each other and exert a synergistic effect in alleviating gouty arthritis, which is specifically manifested by: (1) reducing pain sensitivity; (2) reducing the number of leukocytes and the aggregation of neutrophils in joints; (3) reducing the activity of myeloperoxidase in periarticular tissues; (4) reducing the generation of inflammatory factors CXCL1 and IL-1β; and (5) improving the expression level of anti-inflammatory cytokines IL-10 in joint tissues. In the case where amounts of bacteria used are consistent, compared with an intervention mode of a single BL21 strain or a single LS97 strain, the effect of the compounding of the two bacteria in alleviating and improving gouty arthritis is significantly improved.

### BRIEF DESCRIPTION OF DRAWINGS

FIG. 1 is a statistical chart illustrating the result of pain sensitivity of each group of mice.
FIG. 2 is a statistical chart illustrating the result of a white blood cell count in cavity fluids of the knee joints of each group of mice.
FIG. 3 is a statistical chart illustrating the result of a neutrophil count in cavity fluids of knee joints of each group of mice.
FIG. 4 is a statistical chart illustrating the result of the level of myeloperoxidase (MPO) in knee joint tissues of mice in each group.
FIG. 5 is a statistical chart illustrating the result of the level of chemokine ligand 1 (CXCL1) in knee joint tissues of mice in each group.
FIG. 6 is a statistical chart illustrating the result of the level of interleukin-1β (IL-1β) in knee joint tissues of mice in each group.
FIG. 7 is a statistical chart illustrating the result of the level of interleukin 10 (IL-10) in knee joint tissues of mice in each group.

### DETAILED DESCRIPTION

Technical solutions of the present application are further described below through examples. It is to be understood by those skilled in the art that the examples are intended to facilitate understanding of the present application and are not to be construed as limiting the present application.

The taxonomic name of the BL21 strain involved in the following example is *Bifidobacterium longum* with a deposit number CGMCC No. 10452.

The taxonomic name of the LS97 strain involved in the following example is *Lactobacillus salivarius* with a deposit number CGMCC No. 16922.

The medium involved in the following example is as follows:
MRS medium (g/L): 10 g/L peptone, 10 g/L beef extract, 15 g/L glucose, 15 g/L lactose, 5 g/L yeast powder, 2 g/L diammonium hydrogen citrate, 2.6 g/L K₂PO₄·3H₂O, 0.1 g/L MgSO₄·7H₂O, 0.05 g/L MnSO₄, 1 mL/L polysorbate 80 (Tween 80) and 0.5 g/L cysteine salt.

The bacterial suspension involved in the following example: required strains were inoculated in an MRS liquid medium and cultured for 24 h at 37°C for activation two consecutive times to obtain an activation solution, the activation solution was inoculated in an MRS liquid medium at an inoculum volume of 2% (v/v) and cultured for 24 h at 37°C to obtain a bacterial solution, the bacterial solution was centrifuged at 6000 g for 10 min, and the strains were resuspended with PBS to obtain the bacterial suspension.

The data of the experimental results were statistically analyzed by using graphpad. Compared with a model group, *** represents p < 0.001, ** represents p < 0.01, * represents p < 0.05, and NS. represents no significant difference.

### Example

This example explores the ability of a composite probiotic to alleviate symptoms of a gouty arthritis mouse model.
(1) Test animals: healthy female 8-week-old SPF C57BL/6J mice (48 mice, purchased from Shanghai Laboratory Animal Center). These mice were reared in a controlled environment with room temperature maintained at 22±2°C and a humidity of 55%±5%, following a 12 h light/dark cycle. They had free access to food and water. All experimental procedures involving the mice were in accordance with the ethical guidelines for animal care and use established by Shanghai Laboratory Animal Care and Experimental Center.
(2) Animal grouping: after the above mice were adaptively fed for 1 week, the 48 mice were randomly divided into 6 groups (8 mice for each group): a control group (a Ctl group), a model group (a Treat group), a BL21 group (recorded as an S1 group), an LS97 group (recorded as an S2 group), a composite probiotic group 1 (a BL21+LS97 group with a ratio of viable bacterial count of 1:1, recorded as an S3 group) and a composite probiotic group 2 (an ATCC15707+LS97 group with a ratio of viable bacterial count of 1:1, recorded as an S4 group).
(3) Animal modeling and intervention methods

The mice were anesthetized, and the ankle joints of the right hind limbs of the mice were disinfected with iodophor. The model group and each probiotic intervention group were injected with 200 µL of monosodium urate suspension (concentration: 0.5 mg/mL) in the joint cavity, and the control group was injected with 200 µL of PBS solution in the joint cavity. Moreover, intervention was performed after 15 h of modeling. The control group and the model group were routinely fed, and the probiotic intervention groups were routinely fed and administered with their respective groups of bacterial suspensions (dosage: 1×10¹⁰ CFU/mouse/day) via gavage for intervention for a total of 7 days.

### (4) Indicator detection

(4.1) After the intervention of the probiotics for 7 days, the nociception of the mice was evaluated by using an electronic pressure gauge. Flexion-evoked withdrawal thresholds are used for inferring behavioral responses related to pain, and a progressive pressure is applied on a tail or a hind limb of an animal to evoke a pain response. Once the animal exhibits an apparent pain response, the force of the corresponding pressure applied is recorded. A mouse with gout pain bears a relatively low pressure. The results are represented by average measurement values (in grams).

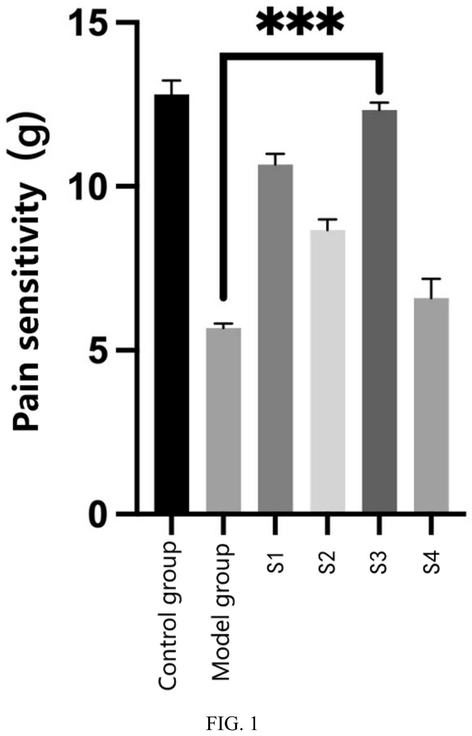

The results are shown in FIG. 1. Compared with the control group, the reactivity of the mice in the model group to mechanical stimulation on inflamed limbs increases. After the intervention of each probiotic group, compared with the model group, the tolerance of the mice treated with the probiotics to high injury reactions increases to varying degrees, and the S3 group is more significant, indicating that the probiotics involved in the present application can alleviate the gout symptoms of the mice.

### (4.2) White blood cell and neutrophil counts in knee joints

After the intervention of the probiotics for 7 days, all the mice were sacrificed through cervical dislocation, knee joint cavities were taken and washed twice with PBS containing 3% bovine serum albumin (5 µL), and cell counting was performed on the effusions in the joint cavities. The number of leukocytes from the joint cavity was measured in a cell counting plate through May-Grünwald stain. The content of the neutrophils was calculated according to a standard curve based on the activity of myeloperoxidase.

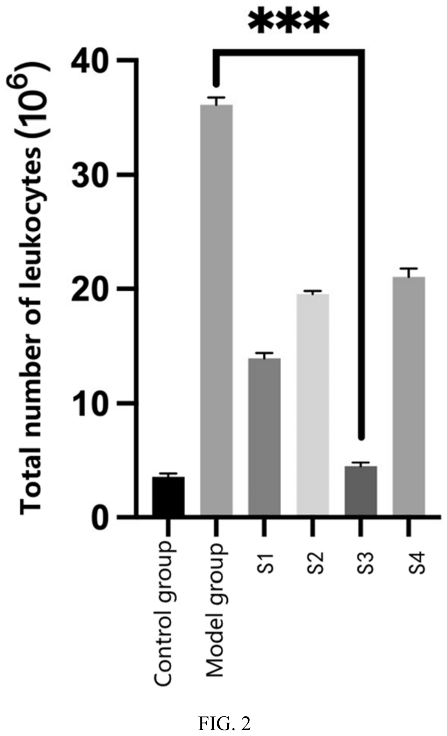

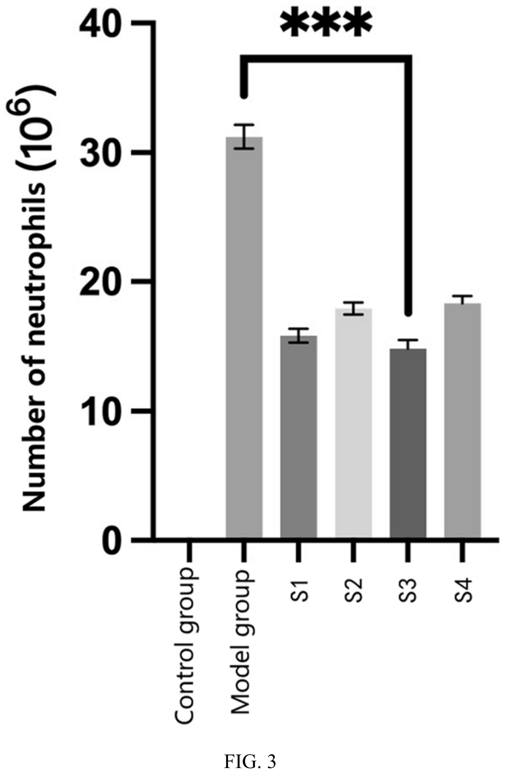

The results are shown in FIGS. 2 and 3. Compared with the control group, the number of leukocytes and the number of neutrophils in the model group increase significantly, and neutrophils are considered to be an important pathological marker of gout inflammation, indicating that the mice in the model group have specific and apparent gout symptoms. After the intervention of each probiotic group, the number of leukocytes and the number of neutrophils decrease to varying degrees, and the S3 group is more significant, indicating that the probiotics involved in the present application can alleviate the gout symptoms of the mice.

### (4.3) Cytokine and chemokine detection

Periarticular tissues of the knees of the mice were collected and homogenized in PBS containing antiproteases, samples were homogenized with 0.05% Tween 20, and levels of myeloperoxidase (MPO), chemokine ligand 1 (CXCL1), interleukin-1β (IL-1β) and interleukin 10 (IL-10) in supernatant obtained after the centrifugation of the homogenate were measured according to ELISA.

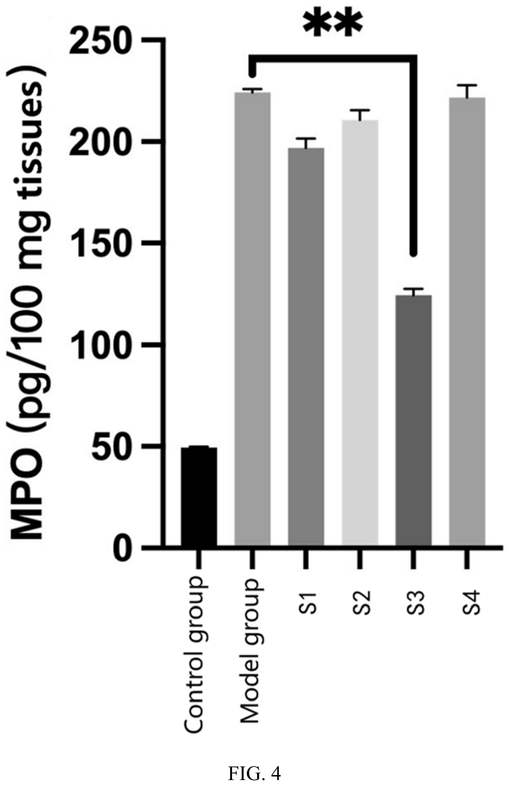

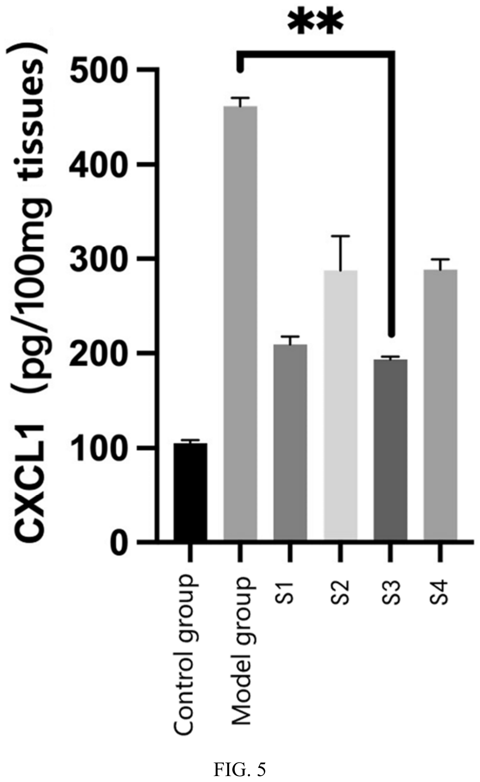

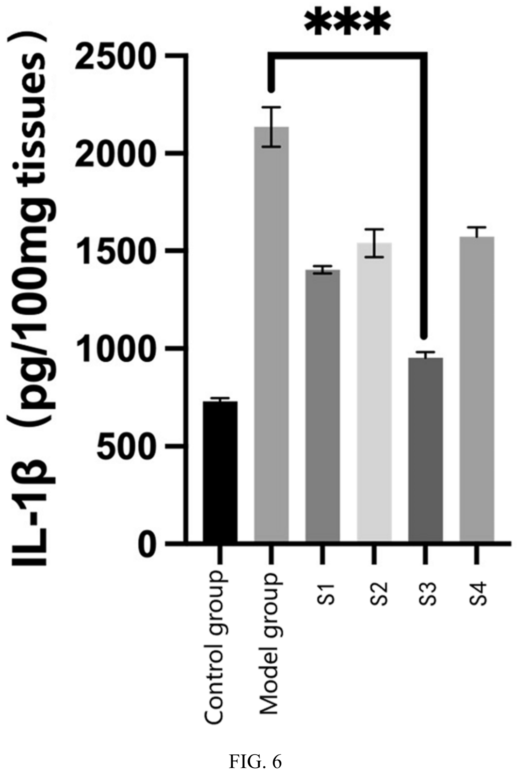

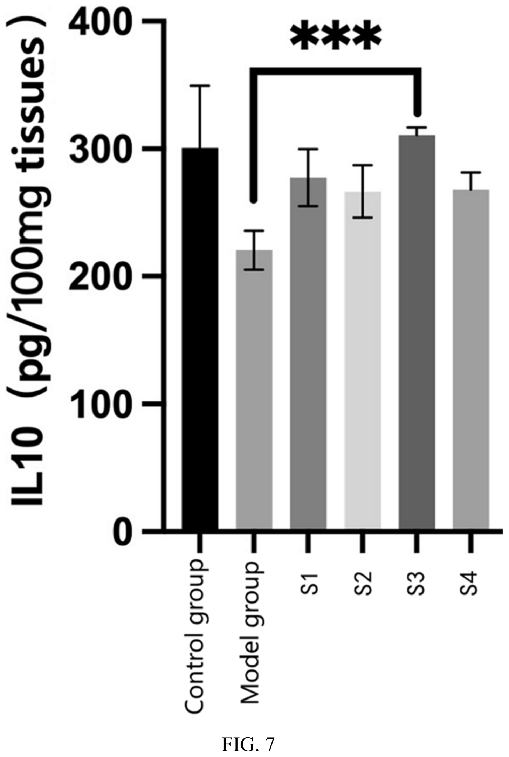

The results are shown in FIGS. 4 to 7, respectively. Compared with the control group, the levels of myeloperoxidase (MPO), chemokine ligand 1 (CXCL1) and interleukin-1β (IL-1β) of the mice in the model group are significantly increased. The high expression of MPO, CXCL1 and IL-1β is related to the aggregation of neutrophils in knee joints, and the level of interleukin 10 (IL-10) of the mice in the model group is decreased. After the intervention of each probiotic group, the levels of myeloperoxidase (MPO), chemokine ligand 1 (CXCL1) and interleukin-1β (IL-1β) are significantly reduced, and the level of interleukin 10 (IL-10) is significantly increased. The increased level of IL-10 is generally related to the spontaneous regression of gout, and the S3 group changes more significantly, indicating that the probiotics involved in the present application can alleviate the gout symptoms of the mice.

The applicant has stated that although the technical solutions of the present application are described through the preceding embodiments, the present application is not limited to the preceding embodiments, which means that the implementation of the present application does not necessarily depend on the preceding embodiments. It is to be understood by those skilled in the art that any improvements made to the present application and equivalent replacements of raw materials of the product, additions of adjuvant ingredients, selections of specific manners, etc. in the present application all fall within the scope of the present application.

Although preferred embodiments of the present application are described above in detail, the present application is not limited to details of the preceding embodiments, and various simple variations can be made to the technical solutions of the present application without departing from the technical concept of the present application. These simple variations are all within the scope of the present application.

Additionally, it is to be noted that if not in collision, specific technical features described in the preceding embodiments can be combined in any suitable manner. To avoid unnecessary repetition, various possible combinations are no longer described in the present application.

## Claims

1. A composite probiotic for alleviating gouty arthritis consisting of a *Bifidobacterium longum* BL21 strain with a deposit number CGMCC No. 10452 and a *Lactobacillus salivarius* LS97 strain with a deposit number CGMCC No. 16922.

2. The composite probiotic for alleviating gouty arthritis according to claim 1, wherein a ratio of a viable bacterial count of the BL21 strain to a viable bacterial count of the LS97 strain is 1:10 to 10:1.

3. A probiotic preparation for alleviating gouty arthritis, comprising the composite probiotic according to claim 1 or 2.

4. The probiotic preparation for alleviating gouty arthritis according to claim 3, wherein a total viable bacterial count in the probiotic preparation is not less than 1×10⁹ CFU/mL or 1×10⁹ CFU/g.

5. The probiotic preparation for alleviating gouty arthritis according to claim 3, wherein a dosage form of the probiotic preparation comprises lyophilized powder, a capsule, a tablet or a granule.

6. The probiotic preparation for alleviating gouty arthritis according to claim 5, wherein the dosage form of the probiotic preparation is the lyophilized powder, and the lyophilized powder is prepared through the following preparation method:
inoculating a BL21 strain and an LS97 strain in mediums, respectively, for activation and fermentation culture to obtain fermentation broths; separately centrifuging the fermentation broths, mixing with a lyoprotectant, and then performing lyophilization to obtain BL21 bacteria powder and LS97 bacteria powder; and mixing the BL21 bacteria powder with the LS97 bacteria powder according to a ratio of viable bacterial count to obtain the probiotic preparation for alleviating gouty arthritis.

7. The probiotic preparation for alleviating gouty arthritis according to claim 6, wherein the lyoprotectant is selected from any one or a combination of at least two of skim milk, gelatin, dextrin, arabic gum, dextran, sodium alginate, polyvinylpyrrolidone, sucrose, lactose, trehalose, sorbitol or xylitol.

8. The probiotic preparation for alleviating gouty arthritis according to claim 6, wherein the BL21 bacteria powder and the LS97 bacteria powder are mixed according to the ratio of the viable bacterial count and mixed with a functional additive; and
the functional additive is selected from any one or a combination of at least two of fructooligosaccharides, galactooligosaccharides, xylooligosaccharides, isomaltooligosaccharides, soybean oligosaccharides, inulin, spirulina, *arthrospira*, Krestin, stachyose, polydextrose, α-whey protein or lactoferrin.

9. A use of the composite probiotic according to claim 1 or 2 or the probiotic preparation according to any one of claims 3 to 8 in the preparation of a product for preventing, alleviating or treating gouty arthritis.

10. The use according to claim 9, wherein the product further comprises an adjuvant.
